# EUROPEAN PATENT APPLICATION

(11) **EP 4 613 264 A1**
(43) Date of publication of application: **10.09.2025**
(21) Application number: 24161727.3
(22) Date of filing: 06.03.2024
(51) Int. Cl.: A61K 9/00, A61K 31/325, A61K 31/7042, A61K 36/45, A61P 9/14

(54) **HYDRO-DISPERSIBLE FLAVONOID COMPOSITION**

(71) Applicant: Laboratorio della Farmacia S.p.A., 30020 Quarto d'Altino VE (IT)
(72) Inventor: Salvagnin, Marzio, 30020 Quarto d'Altino VE (IT); Fratter, Andrea, 30030 Olmo di Martellago VE (IT)
(74) Representative: Perani & Partners S.p.A.

(57) **Abstract**

The present invention relates to a hydro-dispersible oral composition comprising i) active ingredients comprising: at least one flavonoid; ii) an emulsifying vehicle comprising: a caseinate salt, a salt of starch octenyl succinate, and at least one alkalising agent; and the preparation method thereof.

## Description

### FIELD OF THE INVENTION

The present invention relates to a composition comprising active ingredients, in particular flavonoids, and an emulsifying vehicle, preferably for use in treating mild-to-moderate venous insufficiency, congestion of the haemorrhoidal venous plexus, post-surgical oedema, oedema and venous-lymphatic congestion of various aetiologies, and IgE mediated allergies.

### STATE OF THE ART

Flavonoids are a class of polyphenolic substances derived from the secondary metabolism of plants. In plants, they are present in the form of glucosides, in which aglycones can be associated with different sugars to give rise to different flavonoids (e.g., Rutin, Hesperidin). Their name derives from the Latin *Flavus* (blond, yellow) since they colour aqueous solutions yellow.

They are divided into: flavones, derived from 2-phenylchromen-4-one (2-phenyl-1,4-benzopyrone); isoflavones, derived from 3-phenylchromen-4-one (3-phenyl-1,4-benzopyrone); neoflavones, derived from 4-phenylcoumarin (4-phenyl-1,2-benzopyrone).

Flavonoids and the polyphenolic fractions of plants are generally used in medical therapy as vasculo-trophic agents in the course of phlebopathies; they exert an anti-oedemigenous and endothelium-protective action in the course of mild-to-moderate phlebopathies and venous valve incontinence. Thanks to their phenolic functions, flavonoids and polyphenols are effective antioxidants and help to protect the vascular endothelium from oxidative stress that can trigger the formation of atherosclerosis in the arterial intima.

Diosmin and Hesperidin are two of the most widely used substances, as a pairing, in medical therapy for the treatment of mild-to-moderate venous insufficiency and congestion of the haemorrhoidal venous plexus. The anti-inflammatory and anti-allergic action exerted by flavonoid complexes based on Rutin, Hesperidin and Quercetin are also documented. The latter, in particular, is considered an effective antihistamine agent comparable or even superior to sodium cromoglycate.^{[1,2]} The main mechanisms of action of these substances can be connected to COX inhibition and inhibition of the release of inflammatory and allergic mediators such as interleukins and leukotrienes.^{[1]} These substances also intervene by inhibiting histamine release from mast cells and stabilising Th-1 and Th-2 lymphocytes.^{[1-3]}

### Problem of the prior art

Most flavonoids, even if in glucosidic form, are poorly soluble in water below pH=8. Above this value, their solubility exponentially increases due to the salification of the acidic phenolic groups. At these pH values, however, the flavonoids are unstable and undergo a molecular rearrangement, which irreversibly alters the biological properties thereof.^{[4]}

Flavonoids such as Hesperidin are already found to degrade in a simulated enteric environment, irreversibly transforming into more than 50% insoluble chalcones.^{[5]} No less important is the biotransformation phenomenon of flavonoids, especially in glucosidic form, by the enteric microbiome, which expresses enzymes capable of removing sugar (O-deglucosylation) as well as chemically converting aglycones.^{[6,7]}

Due to this known low solubility in water, flavonoids show poor enteric stability and bioaccessibility, which precludes the bioavailability thereof. In fact, Diosmin and Hesperidin tablets with a weight ratio of 9 to 1, in micronised form, so as to increase the contact surface of the active ingredients with the absorbing enteric epithelium and consequently their bioavailability.^{[8-10]}

### SUMMARY OF THE INVENTION

The Applicant has now identified a new form of delivery, capable of solving the problems of the prior art regarding the formulation of poorly soluble and/or unstable substances at physiological pH, such as flavonoids.

A first object of the present invention is a hydro-dispersible oral composition comprising:
i) active ingredients comprising:
   - at least one flavonoid,
ii) an emulsifying vehicle comprising:
   - a caseinate salt,
   - a salt of starch octenyl succinate, and
   - at least one alkalising agent.

The oral composition of the invention is preferably intended for use as a medicament (medical use), or as a food supplement, or as a food for special medical purposes.

A further object of the present invention is a method for preparing the oral composition of the invention comprising the steps of:
*a*) preparing the active ingredients, in powder form;
*b*) preparing the emulsifying vehicle, in powder form;
*c*) mixing the powders of the active ingredients and the vehicle;
*d*) optionally, adding further excipients and/or diluents, in powder form, to the preparation to obtain the oral hydro-dispersible composition,
the method being characterised in that each of the constituent steps is carried out dry and at room temperature.

### Advantages of the invention

The composition of the invention is particularly advantageous with respect to the state-of-the-art solutions, as the emulsifying vehicle allows to:
- increase the hydro-dispersion of the flavonoids in pH conditions below 7, due to the formation of mixed caseinate and starch octenyl succinate micelles which encapsulate the active ingredients;
- encapsulate the flavonoid molecules with high efficiency, ensuring the protection thereof from the gastro-enteric environment and, at the same time, ready hydro-dispersion in the enteric environment;
- prevent the inactivation of the flavonoids at enteric pHs (>8) due to the molecular rearrangement to which they are subjected under these chemical conditions;
- prevent the potential degradation of caseinate salt in the gastric environment (due to the acid pH and the presence of Pepsin), thanks to the inclusion of an alkalising agent capable of bringing the gastric pH between about 5 and 6, values at which Pepsin exerts a reduced proteolytic activity;
- improve the bioavailability of the active ingredients, with respect to the commercially available preparations;
- the emulsifying vehicle also prevents the metabolisation of the flavonoids and polyphenols by saprophytic flora which could impair the biological action thereof, after biotransformation into substances which are no longer active.

### DESCRIPTION OF THE DRAWINGS

Figure 1: Structure of Apigenin and Quercetin.
Figure 2: Structure of Genistein.
Figure 3: Structure of glucosidic flavonoids. Diosmin (left), Hesperidin (centre), Rutin (right)

### DETAILED DESCRIPTION OF THE INVENTION

Below, the Applicant describes the invention in more detail.

### Oral composition

### Active ingredients

The active ingredients of the oral composition of the invention comprise at least one flavonoid (in other words, it comprises one or more molecules belonging to the class of flavonoids).

Flavonoids refers to a group of natural phenolic compounds which are characterised by a phenyl-benzo-γ-pyron-derived structure consisting of two benzene rings. Preferably, the flavonoids suitable for the purposes of the invention are selected from the group consisting of: isoflavones, neoflavonoids (neoflavones and neoflavenes), flavones, flavonols, flavanones, flavanonols, flavanols, anthocyanidins, anthocyanosides, chalcones and mixtures of the above.

Still preferably, the flavonoids conveyed in the composition can be glycosidic flavonoids, non-glycosidic flavonoids or mixtures of the above.

Preferably, the flavonoids contained in the composition are in the form of an extract of a plant of the genus *Citrus* (titrated in flavonoids). Still preferably, the plant of the genus *Citrus* from which the flavonoids are obtainable, for the purposes of the invention, is selected from the group consisting of: *Citrus aurantium, Citrus x sinensis var., Citrus medica, Citrus x paradisi, Citrus x latifolia, Citrus aurantifolia, Citrus japoncia, Citrus lemon, Citrus sinensis, Citrus reticulata, Citrus maxima, Citrus junos* and mixtures of the above.

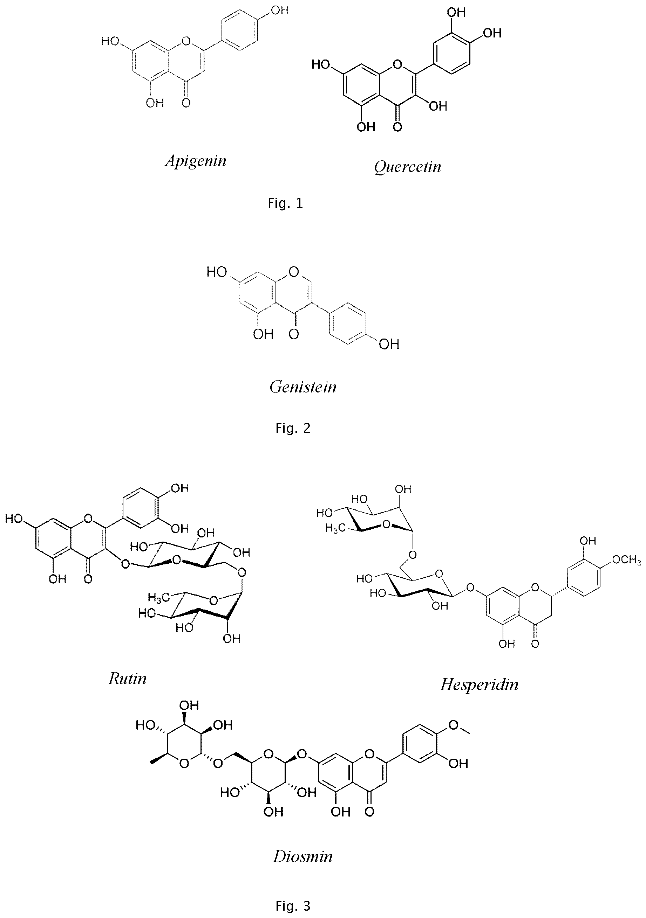

According to a preferred embodiment, the flavonoids of interest are those classifiable as:
- flavones, derivatives of 2-phenylchromen-4-one (2-phenyl-1,4-benzopyrone). Examples of flavonoids belonging to this subclass are Apigenin and Quercetin (Figure 1).
- Isoflavones, derived from 3-phenylchromen-4-one (3-phenyl-1,4-benzopyrone). An example of flavonoids belonging to this subclass is Genistein (Figure 2).
- Neoflavones, derived from 4-phenylcoumarin (4-phenyl-1,2-benzopyrone). Examples of neoflavones in glycosidic form are Diosmin, Hesperidin, Rutin (Figure 3).

Preferably, the at least one flavonoid comprises Diosmin and/or Hesperidin.

Preferably Diosmin and/or Hesperidin are present in the composition in the form of an extract, preferably in dry extract form, of a plant of the genus *Citrus,* preferably of one of the species selected from the group consisting of: *Citrus aurantium, Citrus x sinensis var., Citrus medica, Citrus x paradisi, Citrus x latifolia, Citrus aurantifolia, Citrus japoncia, Citrus lemon, Citrus sinensis, Citrus reticulata, Citrus maxima, Citrus junos* and mixtures of the above.

Still preferably, Diosmin is present in the composition in the form of a dry extract (*Citrus*) titrated in Diosmin in percentages varying between 50% and 100%, preferably between 60% and 90%, still preferably in percentages equal to about 60% and 90% by weight.

Still preferably, Hesperidin is present in the composition in the form of a dry extract (*Citrus*) titrated in Hesperidin in percentages varying between 50% and 100%, preferably between 60% and 90%, still preferably in percentages equal to about 60% and 90% by weight.

Preferably, Diosmin and/or Hesperidin can be used in micronised form or in a form which includes both micronised and amorphous powder.

Still preferably, the at least one flavonoid, in addition to Diosmin and/or Hesperidin, further comprises:
- a flavonoid selected from: Rutin, Quercetin, Apigenin, Genistein, or mixtures of the above, and/or
- Anthocyanosides of *Vaccinium myrtillusl* (bilberry).

It should be noted that Rutin, Quercetin, Apigenin, Genistein are also flavonoids obtainable in extract form, preferably dry extract, from plants of the genus *Citrus.* According to a preferred embodiment, one or more of Rutin, Quercetin, Apigenin, Genistein are preferably in the form of an extract, preferably a dry extract, of one of the species selected from the group consisting of: *Citrus aurantium, Citrus x sinensis var., Citrus medica, Citrus x paradisi, Citrus x latifolia, Citrus aurantifolia, Citrus japoncia, Citrus lemon, Citrus sinensis, Citrus reticulata, Citrus maxima, Citrus junos* and mixtures of the above.

Preferably, anthocyanosides of *V. Myrtillus* are contained in the composition of the invention in the form of an extract of *V*. *Myrtillus.* Still preferably, such an extract is titrated in anthocyanosides comprised between 1 and 36% by weight of the extract.

Still preferably, the flavonoids contained in the composition comprise or consist of Diosmin, and Hesperidin and anthocyanosides of *Vaccinium myrtillus.*

Preferably, the at least one flavonoid (in other words, the one or more flavonoids present in the composition) is comprised between 5 and 40% by weight, preferably between 5% and 35% by weight, preferably between 7% and 30% by weight, preferably between 10% and 30% by weight of the total weight of the composition (w/w).

In accordance with a preferred embodiment, the active ingredients of the composition comprise, in addition to flavonoids and possibly anthocyanosides, also one or more polyphenols.

Polyphenols are intended as a group of photochemical substances with potential favourable effects on health, which contain at least one aromatic nucleus and one or more -OH groups. Polyphenols are commonly categorised as flavonoids, characterised by a C6-C3-C6 structure, and non-flavonoids.

In the case of the present invention, the active ingredients can include polyphenols of Vitis Vinifera, such as Vitexin (flavonoid), Resveratrol (non-flavonoid phenol) and Polydatin (flavonoid).

It should be noted that the polyphenols conveyed in the composition are in the form of *Vitis vinifera* extract, preferably dry extract.

Preferably, the one or more polyphenols contained in the composition are present in concentrations comprised between 1% and 5% by weight of the total weight of the composition.

Preferably, the active ingredients of the oral composition of the invention are active ingredients which are poorly soluble in water.

### Emulsifying vehicle

For the purposes of the invention, an emulsifying vehicle is intended as a set of substances which are capable of promoting the formation of an emulsion, specifically a micellar system, when in contact with an aqueous fluid, be it water or a biological fluid.

The emulsifying vehicle comprises at least:
- a caseinate salt,
- a salt of starch octenyl succinate, and
- at least one alkalising agent.

The caseinate salt and the salt of starch octenyl succinate form mixed micelles in an aqueous environment capable of encapsulating the flavonoid molecules with high efficiency, ensuring the protection thereof from the gastro-enteric environment and, at the same time, ready hydro-dispersion in the enteric environment.

Preferably, the caseinate salt has a counter-cation selected from: sodium, potassium, calcium, ammonium, preferably is sodium.

Still preferably, the caseinate salt is in an amount comprised between 0.5% and 20% by weight, preferably between 0.75% and 15% by weight, preferably between 1% and 10% by weight, preferably between 5% and 10% by weight, of the total weight of the composition (w/w).

Preferably, the salt of starch octenyl succinate is selected from sodium starch octenyl succinate salt, aluminium starch octenyl succinate salt, or mixtures of the above, preferably it is sodium starch octenyl succinate salt.

Still preferably, the salt of starch octenyl succinate is in an amount comprised between 1% and 30% by weight, preferably between 2% and 20% by weight, preferably between 3% and 15% by weight, preferably between 4% and 10% by weight, preferably between 4% and 7% by weight of the total weight of the composition (w/w).

Preferably, the emulsifying vehicle:active ingredient weight ratio is comprised between about 1:3 and 2:1, preferably comprised between 1:2 and 2:1, preferably equal to about 0.5, or 1.1, or 1.4.

According to a preferred embodiment, the alkalising agent is selected from the group consisting of: magnesium oxide, sodium bicarbonate, base L-arginine, a buffer solution of a citrate salt and citric acid, and mixtures of the above.

It should be noted that the citrate salt can be sodium or potassium.

Still preferably, the alkalising agent consists of a buffer of potassium citrate and citric acid. It should be noted that the citrate buffer is titrated in the oral composition in quantities such that the pH of 40 ml of simulated gastric fluid (GSF) is adjusted from a value of about 1.5 to values comprised between about 5.0 and 6.5, preferably 5.5-6.5, and maintained in this range for at least 60 minutes.

As already mentioned among the advantages of the invention, the presence of the alkalising agent, while not *directly* offering an emulsifying action, is a key element of the emulsifying vehicle of the composition of the invention: in fact, the inclusion of an alkalising agent is capable of bringing the gastric pH between 5 and 6, values at which Pepsin exerts only a reduced proteolytic activity. In this pH range, the Caseins are in salt form, a form in which the formation of the mixed micelles described above can occur. In this sense, the alkalising agent contributes to the proper formation of mixed micelles in the gastric environment. Controlling the pH of the stomach also prevents the molecular rearrangement of the flavonoids at pH > 8, which is responsible for altering their biological properties. In this sense, the vehicle not only has emulsifying power, but also stabilising power.

### Physical form of the oral composition

Preferably, the oral composition of the invention is a formulation in solid form. Preferably, the oral composition is in the form of solid preparations to be dissolved in water; or to be taken as such, suitable to give rise to micellar dispersions once disaggregated in gastro-enteric fluids.

A further object of the present invention are aqueous solutions of the oral composition of the invention, obtainable - for example - by dissolution of the composition described in the present patent application in water. It should be noted that the pH of 100 ml of a solution in water of the oral composition is characterised by values comprised between approximately 6.0 and 7.0.

Still preferably, the oral composition of the invention is in powder, granulate or tablet form. Still preferably, when in powder or granulate form, the composition is intended for dissolution in water after administration; when in tablet form, the composition is intended for swallowing as is.

Preferably, one dosage unit of the oral composition comprises Diosmin in amorphous or micronised form, in quantities comprised between 1 and 1000 mg, preferably between 45 and 900 mg.

Preferably, one dosage unit comprises Hesperidin in amorphous or micronised form, in an amount comprised between 1 and 1000 mg, preferably comprised between 25 and 300 mg.

Preferably, a dosage unit comprises Rutin in an amount comprised between 1 and 1000 mg, preferably comprised between 50 and 500 mg.

Preferably, a dosage unit comprises Quercetin in an amount comprised between 1 and 1000 mg, preferably comprised between 50 and 500 mg.

Preferably, one dosage unit comprises an amount comprised between 1 and 72 mg of anthocyanosides of *Vaccinium myrtillus,* preferably in the form of a dry extract titrated in anthocyanosides at 1-36% by weight on the weight of the extract.

According to a preferred embodiment of the invention, the oral composition of the invention further comprises a rheology agent selected from the group consisting of: xanthan gum, hydroxypropyl methylcellulose (HPMC) or mixtures of the above.

It should be noted that, preferably, the oral composition of the invention also comprises any excipients and/or diluents known to the person skilled in the art and suitable for making solid oral dosage forms. By way of example, such excipients and/or diluents are selected from
- diluting/filling agents, e.g., maltodextrins, polyols;
- sliding agents, e.g., amorphous silica;
- flavouring agents;
- sweetening agents, e.g., sucralose, aspartame, acesulfame, stevia rebaudiana glucosides;
- lubricating agents, e.g., magnesium stearate, mono and diglycerides of fatty acids and mixtures of the above.

### Use of the oral composition

The oral composition object by the present invention is intended for use as a medicament (medical use), or as a food supplement, or as a food for special medical purposes.

The oral composition object by the present invention is intended for use:
- in the treatment of mild-to-moderate venous insufficiency;
- in the treatment of the congestion of the haemorrhoidal venous plexus;
- in the treatment of post-surgical oedema;
- in the treatment of the oedema and venous-lymphatic congestion of various aetiologies;
- in the treatment of inflammation of the venous wall, better known as phlebitis;
- in the treatment of IgE-mediated allergies.

### Method for preparing the oral composition

A further object of the invention is the method for preparing the solid composition of the invention comprising the steps of:
*a*) preparing the active ingredients, in powder form;
*b*) preparing the emulsifying vehicle, in powder form;
*c*) mixing the powders of the active ingredients and the emulsifying vehicle;
*d*) optionally, adding further excipients and/or diluents, in powder form, to obtain the oral composition in powder form;
wherein each of the steps constituting the method is carried out dry and at room temperature.

It should be noted that the term "dry" means that each of the steps constituting the method is carried out exclusively by mixing powders with other powders, i.e., without the addition of water, solvents, oils or other liquid/semi-liquid agents which can act as a humectant or solvent.

### EXAMPLES

The Applicant provides the examples hereinafter for merely illustrative and non-limiting purposes of the invention.

### 1. Example 1

### 1.1 Qualitative-quantitative composition.

| **Step** | **Component** | **g/bag** | **g/10 sachet** | **g/100 sachet** |
|---|---|---|---|---|
| A | Citrus flavonoids 60% Hesperidin | 0.500 | 5.00 | 50.00 |
| A | Diosmin >90% | 0.055 | 0.55 | 5.500 |
| A | Bilberry d.e. 1% anthocyanosides | 0.010 | 0.10 | 1.000 |
| B | Clear gum (Sodium Starch Octenyl Succinate salt) | 0.200 | 2.00 | 20.00 |
| C | Xanthan gum | 0.025 | 0.25 | 2.500 |
| B | EMUL AC (sodium caseinate) | 0.150 | 1.50 | 15.00 |
| C | Glucidex 19 (Corn Maltodextrins) | 3.601 | 36.01 | 360.1 |
| E | Sucralose | 0.002 | 0.02 | 0.200 |
| E | Giotti Orange Aroma | 0.075 | 0.75 | 7.500 |
| D | Citric acid | 0.024 | 0.24 | 2.400 |
| D | Potassium citrate | 0.258 | 2.58 | 25.80 |
| F | Amorphous silica | 0.100 | 1.00 | 10.00 |
| | | 5.000 | 50.00 | 500.0 |

### 1.2 Preparation method

Prepare *Step A* by mixing flavonoids and Bilberry extract together for 5 minutes in a nylon bag (balloon). Proceed to combine Sodium Caseinate (EMUL AC) and Starch octenyl succinate (Clear Gum) (*Step B*) previously closely mixed together. Proceed to mix the powder for at least 5 minutes. Add the maltodextrins (Glucidex 19) and xanthan gum (*Step C*) and mix for a further 5 minutes. Add the Potassium Citrate and Citric Acid (citrate buffer) (*Step D*) and mix for a further 5 minutes. Add Sucralose and Aroma (*Step E*) and mix for a further 5 minutes. Finally, add Amorphous Silica (*Step F*) and mix for a further 5 minutes.

### 2. Example 2

### 2.1 Qualitative-quantitative composition

| **Step** | **Component** | **g/bag** | **g/10 sachet** | **g/100 sachet** |
|---|---|---|---|---|
| A | Micronised Hesperidin > 90% | 0.110 | 11.000 | 110.00 |
| A | Micronised Diosmin >90% | 1.000 | 1.100 | 11.00 |
| A | Bilberry d.e. 36% anthocyanosides | 0.010 | 0.100 | 1.000 |
| B | Clear gum (Sodium Starch Octenyl Succinate salt) | 0.250 | 2.500 | 25.00 |
| C | Xanthan gum | 0.1 | 1.000 | 10.00 |
| B | EMUL AC (sodium caseinate) | 0.050 | 0.500 | 5.000 |
| C | Glucidex 19 (Corn Maltodextrins) | 3.018 | 30.18 | 301.8 |
| E | Sucralose | 0.005 | 0.050 | 0.500 |
| E | Giotti Orange Aroma | 0.075 | 0.750 | 7.500 |
| D | Citric acid | 0.024 | 0.240 | 2.400 |
| D | Potassium citrate | 0.258 | 2.580 | 25.80 |
| F | Amorphous silica | 0.100 | 1.000 | 10.00 |
| | | ***5.000*** | ***50.00*** | ***500.0*** |

### 2.2 Preparation method

Prepare *Step A* by mixing flavonoids and Bilberry extract together for 5 minutes in a nylon bag (balloon). Proceed to combine Sodium Caseinate (EMUL AC) and Starch octenyl succinate (Clear Gum) (*Step B*) previously closely mixed together. Proceed to mix the powder for at least 5 minutes. Add the maltodextrins (Glucidex 19) and xanthan gum (*Step* C) and mix for a further 5 minutes. Add the Potassium Citrate and Citric Acid (citrate buffer) (*Step D*) and mix for a further 5 minutes. Add Sucralose and Aroma (*Step E*) and mix for a further 5 minutes. Finally, add Amorphous Silica (*Step F*) and mix for a further 5 minutes.

### 3. Example 3

### 3.1 Qualitative-quantitative composition

| **Step** | **Component** | **g/TAB 1.0 g** | **g/10 TAB 1.0 g** | **g/100 TAB 1.0 g** |
|---|---|---|---|---|
| A | Citrus flavonoids > 90% Micronised Hesperidin | 0.030 | 0.300 | 3.000 |
| A | Diosmin >90% micronised | 0.270 | 2.700 | 27.00 |
| B | Clear gum (Sodium Starch Octenyl Succinate salt) | 0.050 | 0.500 | 5.000 |
| C | Xanthan gum | 0.025 | 0.250 | 2.500 |
| B | EMUL AC (sodium caseinate) | 0.100 | 1.000 | 10.00 |
| C | Calcium monohydrogen phosphate | 0.150 | 1.500 | 15.00 |
| D | Citric acid | 0.024 | 0.24 | 2.400 |
| D | Potassium citrate | 0.258 | 2.58 | 25.80 |
| E | Magnesium stearate | 0.045 | 0.450 | 4.500 |
| E | Sodium carboxymethyl cellulose salt | 0.015 | 0.150 | 1.500 |
| F | Amorphous silica | 0.033 | 0.330 | 3.300 |
| | | ***1.000*** | ***10.00*** | ***100.0*** |

### 3.2 Preparation method

Prepare *Step A* by mixing the flavonoids together for 5 minutes in a nylon bag (balloon). Proceed to combine Sodium Caseinate (EMUL AC) and Starch octenyl succinate (Clear Gum) (*Step B*) previously closely mixed together. Proceed to mix the powder for at least 5 minutes. Add the xanthan gum and Calcium monohydrogen phosphate (*Step C*) and mix for a further 5 minutes. Add the Potassium Citrate and Citric Acid (citrate buffer) (*Step D*) and mix for a further 5 minutes. Add Magnesium Stearate and Carboxymethylcellulose (*Step E*) and mix for a further 5 minutes. Finally, add Amorphous Silica (*Step F*) and mix for a further 5 minutes. Proceed to compress.
Weight (average of 10 tablets): 1.02 g
Hardness (average of 10 tablets): 16,3 Kg

### REFERENCES

1. Ribeiro D, Freitas M, Tomé SM, Silva AM, Laufer S, Lima JL, Femandes E. Flavonoids inhibit COX-1 and COX-2 enzymes and cytokine/chemokine production in human whole blood. Inflammation. 2015 Apr;38(2):858-70. doi: 10.1007/s10753-014-9995-x. PMID: 25139581.Banik S, Halder S, Sato H, Onoue S.
2. Mlcek J, Jurikova T, Skrovankova S, Sochor J. Quercetin and Its Anti-Allergic Immune Response. Molecules. 2016 May 12;21(5):623. doi: 10.3390/molecules21050623. PMID: 27187333; PMCID: PMC6273625.
3. Weng Z, Zhang B, Asadi S, Sismanopoulos N, Butcher A, Fu X, Katsarou-Katsari A, Antoniou C, Theoharides TC. Quercetin is more effective than cromolyn in blocking human mast cell cytokine release and inhibits contact dermatitis and photosensitivity in humans. PLoS One. 2012;7(3):e33805. doi: 10.1371/journal.pone.0033805. Epub 2012 Mar 28. PMID: 22470478; PMCID: PMC3314669.
4. Mendel Friedman and Hella S. Jürgens. Effect of pH on the Stability of Plant Phenolic Compounds. Journal of Agricultural and Food Chemistry 2000 48 (6), 2101-2110. DOI: 10.1021/jf990489j.
5. Giacomo Pepe et al. Bioavailable Citrus sinensis Extract: Polyphenolic Composition and Biological Activity. Molecules 2017, 22, 623; doi:10.3390/molecules22040623.
6. Braune A, Blaut M. Bacterial species involved in the conversion of dietary flavonoids in the human gut. Gut Microbes. 2016;7(3):216-234. doi:10.1080/19490976.2016.1158395.
7. Riva Alessandra et al. Conversion of Rutin, a Prevalent Dietary Flavonol, by the Human Gut Microbiota. Front. Microbiol., 21, 11, December 2020 |https://doi.org/10.3389/fmicb.2020.585428
8. Zhao J, Yang J, Xie Y. Improvement strategies for the oral bioavailability of poorly water-soluble flavonoids: An overview. Int J Pharm. 2019 Oct 30;570:118642. doi: 10.1016/j.ijpharm.2019.118642. Epub 2019 Aug 22. PMID: 31446024.
9. Di Lorenzo C, Colombo F, Biella S, Stockley C, Restani P. Polyphenols and Human Health: The Role of Bioavailability. Nutrients. 2021 Jan 19;13(1):273. doi: 10.3390/nu13010273. PMID: 33477894; PMCID: PMC7833401.
10. Amato C. Advantage of a micronized flavonoidic fraction (Daflon 500 mg) in comparison with a nonmicronized diosmin. Angiology. 1994 Jun;45(6 Pt 2):531-6. PMID: 8203783.
11. Karwal R, Garg T, Rath G, Markandeywar TS. Current Trends in Self-Emulsifying Drug Delivery Systems (SEDDSs) to Enhance the Bioavailability of Poorly Water-Soluble Drugs. Crit Rev Ther Drug Carrier Syst. 2016;33(1):1-39. doi: 10.1615/CritRevTherDrugCarrierSyst.v33.i1.20. PMID: 27279337.
12. Chaumeil JC. Micronization: a method of improving the bioavailability of poorly soluble drugs. Methods Find Exp Clin Pharmacol. 1998 Apr;20(3):211-5. PMID: 9646283.

## Claims

1. A hydro-dispersible oral composition comprising
i) active ingredients comprising:
- at least one flavonoid,
ii) an emulsifying vehicle comprising:
- a caseinate salt,
- a salt of starch octenyl succinate, and
- at least one alkalising agent.

2. The composition according to claim 1, wherein the at least one flavonoid comprises Diosmin and/or Hesperidin.

3. The composition according to claim 2, wherein the at least one flavonoid further comprises
- a flavonoid selected from Rutin, Quercetin, Apigenin, Genistein, or mixtures of the above, and/or
- anthocyanosides of *Vaccinium myrtillus.*

4. The composition according to any one of claims from 1 to 3, wherein the active ingredients (i) further comprise polyphenols of *Vitis vinifera.*

5. The composition according to any one of claims from 1 to 4, further comprising a rheology agent selected from the group consisting of: xanthan gum, hydroxypropyl methylcellulose (HPMC), or mixtures of the above.

6. The composition according to any one of claims from 1 to 5 wherein the at least one flavonoid is in an amount between 5% and 40% by weight, preferably between 5% and 35% by weight, preferably between 7% and 30%, preferably between 10% and 30% by weight of the total weight of the composition (w/w).

7. The composition according to any one of claims from 1 to 6, wherein the caseinate salt is in an amount between 0.5% and 20% by weight, preferably between 0.75% and 15% by weight, preferably between 1% and 10% by weight, preferably between 5% and 10% by weight, of the total weight of the composition (w/w).

8. The composition according to any one of claims from 1 to 7, wherein the salt of starch octenyl succinate is in an amount between 1% and 30% by weight, preferably between 2% and 20% by weight, preferably between 3% and 15% by weight, preferably between 4% and 10% by weight of the total weight of the composition (w/w).

9. The composition according to any one of claims from 1 to 8, wherein the alkalising agent is selected from the group consisting of: magnesium oxide, sodium bicarbonate, base L-arginine, and a buffer solution of a citrate salt and citric acid.

10. The composition according to any of claims from 1 to 9, in powder, granulate or tablet form.

11. The composition according to any one of claims from 1 to 10, for use as a medicament, or as a food supplement, or as a food for special medical purposes.

12. The composition according to any one of claims from 1 to 11, for use:
- in the treatment of mild-to-moderate venous insufficiency;
- in the treatment of congestion of the haemorrhoidal venous plexus;
- in the treatment of post-surgical oedema;
- in the treatment of the oedema and venous-lymphatic congestion of various aetiologies;
- in the treatment of phlebitis;
- in the treatment of IgE-mediated allergies.

13. Method for preparing the oral composition according to any one of claims from 1 to 10 comprising the steps of:
*a*) preparing the active ingredients, in powder form;
*b*) preparing the emulsifying vehicle, in powder form;
*c*) mixing the powders of the active ingredients and the emulsifying vehicle;
*d*) optionally, adding further excipients and/or diluents, in powder form, to the preparation to obtain the oral composition in powder form;
wherein each of the steps constituting the method is carried out dry and at room temperature.
